# EUROPEAN PATENT APPLICATION

(11) **EP 4 819 513 A1**
(43) Date of publication of application: **07.10.2026**
(21) Application number: 24895359.8
(22) Date of filing: 25.03.2024
(51) Int. Cl.: H04B 17/309

(54) **GENERATION METHOD FOR LIVING BODY DETECTION MODEL, LIVING BODY DETECTION METHOD AND RELATED DEVICE**

(30) Priority: 27.11.2023 CN 202311598791
(71) Applicant: Amlogic (Shanghai) Co., Ltd., Shanghai 201203 (CN)
(72) Inventor: ZHANG, Xiaofeng, Shanghai 201203 (CN)
(74) Representative: Maiwald GmbH
(86) International application number: PCT/CN2024/083653
(87) International publication number: WO 2025/112247

(57) **Abstract**

The present disclosure relates to a generation method for a living body detection model, living body detection method, and related device, wherein the generation method for a living body detection model, comprises: obtaining channel state information of multi-channel received signals of a Wi-Fi receiving device based on a preset collecting period, a received signal of each channel comprising a plurality of subcarriers; performing conjugate multiplication processing on the channel state information of any two of the multi-channel received signals obtained in each collecting period, respectively, to obtain a plurality of corresponding conjugate multiplication result matrices; extracting amplitude information and phase information of the plurality of subcarriers from the plurality of conjugate multiplication result matrices, respectively; obtaining a plurality of corresponding living body detection training data based on the amplitude information and the phase information of the plurality of subcarriers, to form a living body detection training dataset; and performing learning training using the living body detection training data in the living body detection training dataset to obtain a corresponding living body detection model. The solutions provided by the embodiments of the present disclosure can improve the accuracy of living body detection.

## Description

### TECHNICAL FIELD

The present disclosure relates to the technical field of living body detection, and in particular, to a generation method for a living body detection model, a living body detection method, and related device.

### BACKGROUND

With the development of wireless communication technology, living body detection has been widely applied in fields such as localization within environments under detection, personnel intrusion detection, home medical monitoring, and novel human-machine interaction interfaces. Common living body detection methods include those based on vision, sensors, Wi-Fi, or the like.

Wi-Fi-based living body detection methods have been widely applied due to their non-invasive feature. Wi-Fi-based living body detection methods primarily utilize signal features including Received Signal Strength Indicator (RSSI) and Channel State Information (CSI) to perform living body detection. Compared to RSSI, Channel State Information contains richer multi-path information, which can provide higher accuracy and resolution for living body detection.

However, the accuracy of existing Wi-Fi-based living body detection methods still needs to be improved.

### SUMMARY

Some embodiments of the present disclosure provide a generation method for a living body detection model, a living body detection method, and related device, to help improve the performance of the living body detection model, thereby further improving the accuracy of living body detection.

In some embodiments, a generation method for a living body detection model, comprising: obtaining channel state information of multi-channel received signals of a Wi-Fi receiving device based on a preset collecting period, a received signal of each channel comprising a plurality of subcarriers; performing conjugate multiplication processing on the channel state information of any two of the multi-channel received signals obtained in each collecting period, respectively, to obtain a plurality of corresponding conjugate multiplication result matrices; extracting amplitude information and phase information of the plurality of subcarriers from the plurality of conjugate multiplication result matrices, respectively; obtaining a plurality of corresponding living body detection training data based on the amplitude information and the phase information of the plurality of subcarriers, to form a living body detection training dataset; and performing learning training using the living body detection training data in the living body detection training dataset to obtain a corresponding living body detection model.

The technical solutions of embodiments of the present disclosure have the following advantages: in some embodiments, a generation method for a living body detection model, a living body detection method, and related device provided by embodiments of the present disclosure, wherein the generation method for a living body detection model, by first performing conjugate multiplication processing on the channel state information of any two of the multi-channel received signals of the Wi-Fi receiving device obtained in each collecting period, eliminates the same phase errors generated by the channel state information of different received signals; by then extracting the amplitude information and the phase information of the plurality of subcarriers from the plurality of conjugate multiplication result matrices, respectively, and obtaining a plurality of corresponding living body detection training data based on the amplitude information and the phase information of the plurality of subcarriers, blind zones in changes of amplitude information or phase information of motion at certain positions within the environment to be detected can be eliminated, which helps improve the accuracy of the living body detection training data, thereby helping improve the performance of the living body detection model generated, and further helping improve the accuracy of living body detection.

In some embodiments, the living body detection training data comprises: a living body respiration frequency value calculated based on a phase difference information correlation matrix of the plurality of subcarriers; a maximum eigenvalue, a second largest eigenvalue, and an entropy of eigenvalues after normalization of an amplitude information correlation coefficient matrix of the plurality of subcarriers; an energy value of a first principal component, an energy value of a second principal component, and an energy value of a third principal component, a ratio between a variance of the first principal component and a differential value of an eigenvector corresponding to the variance of the first principal component, a ratio between a variance of the second principal component and a differential value of an eigenvector corresponding to the variance of the second principal component, a ratio between a variance of the third principal component and a differential value of an eigenvector corresponding to the variance of the third principal component, and a kurtosis value of the second principal component, obtained by performing principal component analysis processing on the amplitude information of the plurality of subcarriers; a maximum eigenvalue and a second largest eigenvalue after normalization of a phase difference information correlation coefficient matrix of the plurality of subcarriers; a standard deviation of maximum eigenvalues after normalization of amplitude information correlation coefficient matrices of the plurality of subcarriers corresponding to a current collecting period and previous (N-1) collecting periods; and a standard deviation of second largest eigenvalues after normalization of phase difference information correlation coefficient matrices of the plurality of subcarriers corresponding to the current collecting period and the previous (N-1) collecting periods.

In some embodiments, a value range of N is from 5 times to 10 times.

In some embodiments, a time length of the collecting period is from 1s to 3s.

In some embodiments, the living body detection model comprises a support vector machine classifier.

In some embodiments, the living body comprises at least one of a human body and an animal body having a respiration frequency close to the respiration frequency of the human body.

In some embodiments, a generation module for a living body detection model, comprising: a first obtaining sub-module, configured to obtain channel state information of multi-channel received signals of a Wi-Fi receiving device based on a preset collecting period, a received signal of each channel comprising a plurality of subcarriers; a conjugate multiplication sub-module, configured to perform conjugate multiplication processing on the channel state information of any two of the multi-channel received signals obtained in each collecting period, respectively, to obtain a plurality of corresponding conjugate multiplication result matrices; an information extraction sub-module, configured to extract amplitude information and phase information of the plurality of subcarriers from the plurality of conjugate multiplication result matrices, respectively; a data obtaining sub-module, configured to obtain a plurality of corresponding living body detection training data based on the amplitude information and the phase information of the plurality of subcarriers, to form a living body detection training dataset; and a model training sub-module, configured to perform learning training using the living body detection training data in the living body detection training dataset to obtain a corresponding living body detection model.

In some embodiments, the living body detection training data obtained by the data obtaining sub-module comprises: a living body respiration frequency value calculated based on a phase difference information correlation matrix of the plurality of subcarriers; a maximum eigenvalue, a second largest eigenvalue, and an entropy of eigenvalues after normalization of an amplitude information correlation coefficient matrix of the plurality of subcarriers; an energy value of a first principal component, an energy value of a second principal component, and an energy value of a third principal component, a ratio between a variance of the first principal component and a differential value of an eigenvector corresponding to the variance of the first principal component, a ratio between a variance of the second principal component and a differential value of an eigenvector corresponding to the variance of the second principal component, a ratio between a variance of the third principal component and a differential value of an eigenvector corresponding to the variance of the third principal component, and a kurtosis value of the second principal component, obtained by performing principal component analysis processing on the amplitude information of the plurality of subcarriers; a maximum eigenvalue and a second largest eigenvalue after normalization of a phase difference information correlation coefficient matrix of the plurality of subcarriers; a standard deviation of maximum eigenvalues after normalization of amplitude information correlation coefficient matrices of the plurality of subcarriers corresponding to a current collecting period and previous (N-1) collecting periods; and a standard deviation of second largest eigenvalues after normalization of phase difference information correlation coefficient matrices of the plurality of subcarriers corresponding to the current collecting period and the previous (N-1) collecting periods.

In some embodiments, a value range of N is from 5 times to 10 times.

In some embodiments, a time length of the collecting period is from 1s to 3s.

In some embodiments, the living body detection model comprises a support vector machine classifier.

In some embodiments, the living body comprises at least one of a human body and an animal body having a respiration frequency close to the respiration frequency of the human body.

In some embodiments, a living body detection method, comprising: obtaining channel state information of multi-channel received signals of a Wi-Fi receiving device in a current detection period; and inputting the channel state information of the multi-channel received signals of the Wi-Fi receiving device obtained in the current detection period into the living body detection model generated by the generation method for a living body detection model according to any one of the above, to obtain a corresponding living body detection result.

In some embodiments, the living body detection method further comprising: obtaining, using the living body detection model, an entropy of eigenvalues after normalization of an amplitude information correlation coefficient matrix of the plurality of subcarriers in the current detection period and an entropy of eigenvalues after normalization of amplitude information correlation coefficient matrices of the plurality of subcarriers in previous (M-1) detection periods; and outputting, using the living body detection model, a detection result indicating no living body exists in an environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold.

In some embodiments, a value range of M is from 5 times to 10 times.

In some embodiments, a living body detection module, comprising: an obtaining sub-module, configured to obtain channel state information of multi-channel received signals of a Wi-Fi receiving device in a current detection period; and a detection sub-module, configured to input the channel state information of the multi-channel received signals of the Wi-Fi receiving device obtained in the current detection period into the living body detection model generated by the generation method for a living body detection model according to any one of the above, to obtain a corresponding living body detection result.

In some embodiments, the received signal of each channel comprises a plurality of subcarriers; the detection sub-module is further configured to obtain, using the living body detection model, an entropy of eigenvalues after normalization of an amplitude information correlation coefficient matrix of the plurality of subcarriers in the current detection period and an entropy of eigenvalues after normalization of amplitude information correlation coefficient matrices of the plurality of subcarriers in previous (M-1) detection periods; and output, using the living body detection model, a detection result indicating no living body exists in an environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold.

In some embodiments, a value range of M is from 5 times to 10 times.

In some embodiments, a chip, wherein the generation module for a living body detection model according to any one of the above or the living body detection module according to any one of the above is integrated on the chip.

In some embodiments, an electronic device, comprising at least one memory and at least one processor, wherein the memory stores one or more computer instructions, and the one or more computer instructions are executed by the processor to implement the generation method for a living body detection model according to any one of the above or the living body detection method according to any one of the above.

In some embodiments, a storage medium, wherein the storage medium stores one or more computer instructions, and the one or more computer instructions are configured to implement the generation method for a living body detection model according to any one of the above or the living body detection method according to any one of the above.

### BRIEF DESCRIPTION OF THE DRAWINGS

The accompanying drawings, which comprise a part of this specification, illustrate several embodiments and, together with the description, serve to explain the principles and features of the disclosed embodiments. In the drawings:
FIG. 1 shows a schematic flow diagram of a generation method for a living body detection model provided by an embodiment of the present disclosure.
FIG. 2 shows a schematic structural diagram of a generation module for a living body detection model provided by an embodiment of the present disclosure.
FIG. 3 shows a schematic flow diagram of a living body detection method provided by an embodiment of the present disclosure.
FIG. 4 shows a schematic structural diagram of a living body detection module provided by an embodiment of the present disclosure.
FIG. 5 shows an optional hardware structure diagram of an electronic device provided by an embodiment of the present disclosure.

### DETAILED DESCRIPTION

Currently, the accuracy of existing Wi-Fi-based living body detection methods still needs to be improved.

To solve the above technical problem, the embodiments of the present disclosure provide a generation method for a living body detection model, comprising: obtaining channel state information of multi-channel received signals of a Wi-Fi receiving device based on a preset collecting period, a received signal of each channel comprising a plurality of subcarriers; performing conjugate multiplication processing on the channel state information of any two of the multi-channel received signals obtained in each collecting period, respectively, to obtain a plurality of corresponding conjugate multiplication result matrices; extracting amplitude information and phase information of the plurality of subcarriers from the plurality of conjugate multiplication result matrices, respectively; obtaining a plurality of corresponding living body detection training data based on the amplitude information and the phase information of the plurality of subcarriers, to form a living body detection training dataset; and performing learning training using the living body detection training data in the living body detection training dataset to obtain a corresponding living body detection model.

Wherein the generation method for a living body detection model, by first performing conjugate multiplication processing on the channel state information of any two of the multi-channel received signals of the Wi-Fi receiving device obtained in each collecting period, eliminates the same phase errors generated by the channel state information of different received signals; by then extracting the amplitude information and the phase information of the plurality of subcarriers from the plurality of conjugate multiplication result matrices, respectively, and obtaining a plurality of corresponding living body detection training data based on the amplitude information and the phase information of the plurality of subcarriers, blind zones in changes of amplitude information or phase information of motion at certain positions within the environment to be detected can be eliminated, which helps improve the accuracy of the living body detection training data, thereby helping improve the performance of the living body detection model generated, and further helping improve the accuracy of living body detection.

To make the above purposes, features, and advantages of the present disclosure more apparent and easier to understand, a detailed description of specific embodiments of the present disclosure will be given below in conjunction with the accompanying drawings.

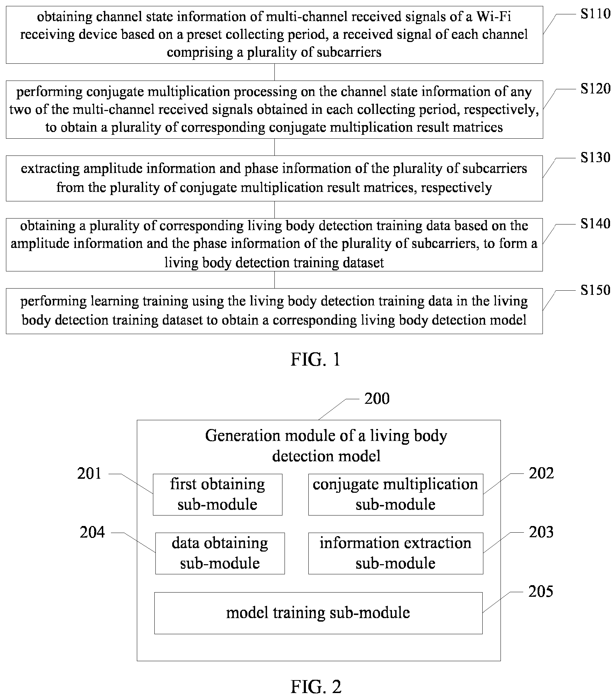

FIG. 1 shows a schematic flow diagram of a generation method for a living body detection model provided by an embodiment of the present disclosure. Referring to FIG. 1, a generation method for a living body detection model may specifically comprise the following steps:
Step S110: obtaining channel state information of multi-channel received signals of a Wi-Fi receiving device based on a preset collecting period, a received signal of each channel comprising a plurality of subcarriers;
Step S120: performing conjugate multiplication processing on the channel state information of any two of the multi-channel received signals obtained in each collecting period, respectively, to obtain a plurality of corresponding conjugate multiplication result matrices;
Step S130: extracting amplitude information and phase information of the plurality of subcarriers from the plurality of conjugate multiplication result matrices, respectively;
Step S 140: obtaining a plurality of corresponding living body detection training data based on the amplitude information and the phase information of the plurality of subcarriers, to form a living body detection training dataset; and
Step S150: performing learning training using the living body detection training data in the living body detection training dataset to obtain a corresponding living body detection model.

Referring still to FIG. 1, in step S110, obtaining channel state information of multi-channel received signals of a Wi-Fi receiving device based on a preset collecting period, a received signal of each channel comprising a plurality of subcarriers.

Obtaining the channel state information of the multi-channel received signals of the Wi-Fi receiving device based on the preset collecting period, with a received signal of each channel comprising a plurality of subcarriers, provides a basis for subsequently performing conjugate multiplication processing on the channel state information of any two of the multi-channel received signals obtained in each collecting period, respectively, to obtain a plurality of corresponding conjugate multiplication result matrices.

In some embodiments, a Wi-Fi transmitting device transmits a Wi-Fi signal using one transmitting antenna, and a Wi-Fi receiving device simultaneously receives the Wi-Fi signal transmitted by the Wi-Fi transmitting device using a plurality of receiving antennas.

In an embodiment of the present disclosure, a Wi-Fi transmitting device transmits a Wi-Fi signal using one transmitting antenna, and a Wi-Fi receiving device simultaneously receives the Wi-Fi signal transmitted by the Wi-Fi transmitting device using two receiving antennas.

The Wi-Fi signal is a Channel Frequency Response (CFR) data packet. Accordingly, in an embodiment of the present disclosure, the Wi-Fi transmitting device transmits a channel frequency response data packet using one transmitting antenna, and the Wi-Fi receiving device simultaneously receives the channel frequency response data packet transmitted by the Wi-Fi transmitting device using two receiving antennas.

In an embodiment of the present disclosure, the received signal of each channel comprises a plurality of subcarriers. Accordingly, the channel state information of the received signal of each channel is the channel state information of a plurality of subcarriers of each receiving antenna of the Wi-Fi receiving device.

The channel state information of the multi-channel received signals of the Wi-Fi receiving device reflects the amplitude and phase information of a plurality of subcarriers of each receiving antenna of the Wi-Fi receiving device. The position of a living body and the movement direction of the living body, or the like, may cause changes in the amplitude and phase of a plurality of subcarriers of each receiving antenna of the Wi-Fi receiving device. Therefore, obtaining the channel state information of the multi-channel received signals of the Wi-Fi receiving device based on the preset collecting period enables the realization of living body detection.

The collecting period of the channel state information of the multi-channel received signals of the Wi-Fi receiving device may be set by a person skilled in the art based on the collecting needs of the channel state information of the multi-channel received signals of the Wi-Fi receiving device.

It is understandable that the collecting period of the channel state information of the multi-channel received signals of the Wi-Fi receiving device should be neither too long nor too short. If the collecting period of the channel state information of the multi-channel received signals of the Wi-Fi receiving device is too long, the real-time performance of the channel state information of the multi-channel received signals of the Wi-Fi receiving device in the time dimension will be reduced, thereby reducing the real-time performance of subsequent data processing and increasing the subsequent amount of calculation; if the collecting period of the channel state information of the multi-channel received signals of the Wi-Fi receiving device is too short, the complete channel state information of the multi-channel received signals of the Wi-Fi receiving device in the time dimension cannot be obtained, which will accordingly reduce the accuracy of the subsequently obtained data, and further affect the accuracy of the living body detection model generated. Therefore, in an embodiment of the present disclosure, a time length of the collecting period is from 1s to 3s.

Referring still to FIG. 1, in step S120: performing conjugate multiplication processing on the channel state information of any two of the multi-channel received signals obtained in each collecting period, respectively, to obtain a plurality of corresponding conjugate multiplication result matrices;
Performing conjugate multiplication processing on the channel state information of any two of the multi-channel received signals obtained in each collecting period, respectively, to obtain a plurality of corresponding conjugate multiplication result matrices, provides a basis for subsequently extracting the amplitude information and the phase information of the plurality of subcarriers from the plurality of conjugate multiplication result matrices, respectively.

Performing conjugate multiplication processing on the channel state information of any two of the multi-channel received signals of the Wi-Fi receiving device obtained in each collecting period can eliminate the same random phase errors generated by the channel state information of the plurality of subcarriers of different receiving antennas, thereby improving the accuracy of the channel state information of the plurality of subcarriers of the receiving antennas of the Wi-Fi receiving device; subsequently extracting the amplitude information and the phase information of the plurality of subcarriers from the plurality of conjugate multiplication result matrices, respectively, can improve the accuracy of the amplitude information and the phase information of the plurality of subcarriers obtained.

**In** the case where the Wi-Fi transmitting device transmits channel frequency response data packets using P transmitting antennas, the Wi-Fi receiving device simultaneously receives the channel frequency response data packets transmitted by the Wi-Fi transmitting device using Q receiving antennas, and the received signal of each of the receiving antennas comprises S subcarriers, a channel frequency response matrix composed of L channel frequency response data packets transmitted within a preset collecting period may be expressed as:
$H = \left[\begin{matrix}{H}^{1} \\ {H}^{2} \\ ⋮ \\ {H}^{L}\end{matrix}\right]$ (1)

Wherein *H* represents the channel frequency response matrix, *H^{k}* represents the *k -th* channel frequency response data packet, and *k* is an integer greater than or equal to 1 and less than or equal to L.

Accordingly, each channel frequency response data packet transmitted by each transmitting antenna of the Wi-Fi transmitting device to each receiving antenna of the Wi-Fi receiving device simultaneously may be expressed as:
${H}_{ij}^{t} = \left[{H}_{ij}^{t}\left(1\right) {H}_{ij}^{t}\left(2\right) ⋯ {H}_{ij}^{t}\left(s\right) ⋯ {H}_{ij}^{t}\left(S\right)\right]$ (2)

Wherein ${H}_{ij}^{t}$ represents the *t -th* channel frequency response data packet transmitted by the *i -th* transmitting antenna of the Wi-Fi transmitting device to the *j -th* receiving antenna of the Wi-Fi receiving device; ${H}_{ij}^{t} \left(s\right)$ represents the channel frequency response data of the s -th subcarrier of the *j*-th receiving antenna of the Wi-Fi receiving device in the *t -th* channel frequency response data packet transmitted by the *i -th* transmitting antenna of the Wi-Fi transmitting device to the *j*-th receiving antenna of the Wi-Fi receiving device; and s is an integer greater than or equal to 1 and less than or equal to *S*.

Accordingly, the *t*-th channel frequency response data packet transmitted by the P transmitting antennas of the Wi-Fi transmitting device to the Q receiving antennas of the Wi-Fi receiving device simultaneously may be expressed as:
${H}^{t} = \left[\begin{matrix}{H}_{11}^{t} & {H}_{12}^{t} & ⋯ & {H}_{1 j}^{t} & ⋯ & {H}_{1 Q}^{t} \\ {H}_{21}^{t} & {H}_{22}^{t} & ⋯ & {H}_{2 j}^{t} & ⋯ & {H}_{2 Q}^{t} \\ ⋮ & ⋮ & & ⋮ & & ⋮ \\ {H}_{i 1}^{t} & {H}_{i 2}^{t} & ⋱ & {H}_{ij}^{t} & ⋱ & {H}_{iQ}^{t} \\ ⋮ & ⋮ & & ⋮ & & ⋮ \\ {H}_{P 1}^{t} & {H}_{P 2}^{t} & ⋯ & {H}_{Pj}^{t} & ⋯ & {H}_{PQ}^{t}\end{matrix}\right]$ (3)

Wherein *H*^{t} represents the channel frequency response data matrix transmitted by the P transmitting antennas of the Wi-Fi transmitting device to the Q receiving antennas of the Wi-Fi receiving device in the *t*-th channel frequency response data packet.

Accordingly, performing conjugate multiplication processing on the channel state information of any two of the multi-channel received signals obtained in each collecting period, respectively, to obtain a plurality of corresponding conjugate multiplication result matrices may be expressed as:
${H}_{cm} = \left[\begin{matrix}{H}_{11}^{1} conj \left({H}_{12}^{1}\right) & {H}_{21}^{1} conj \left({H}_{22}^{1}\right) & ⋯ & {H}_{P 1}^{1} conj \left({H}_{P 2}^{1}\right) & ⋯ \\ {H}_{11}^{2} conj \left({H}_{12}^{2}\right) & {H}_{21}^{2} conj \left({H}_{22}^{2}\right) & ⋯ & {H}_{P 1}^{2} conj \left({H}_{P 2}^{2}\right) & ⋯ \\ ⋮ & ⋮ & ⋱ & ⋮ & ⋱ \\ {H}_{11}^{L} conj \left({H}_{12}^{L}\right) & {H}_{21}^{L} conj \left({H}_{22}^{L}\right) & ⋯ & {H}_{P 1}^{L} conj \left({H}_{P 2}^{L}\right) & ⋯\end{matrix}\right]$ (4)

Wherein conj(.) represents the conjugate operation.

In an embodiment of the present disclosure, a Wi-Fi transmitting device transmits a Wi-Fi signal using one transmitting antenna, and a Wi-Fi receiving device simultaneously receives the Wi-Fi signal transmitted by the Wi-Fi transmitting device using two receiving antennas. Accordingly, the conjugate multiplication result matrix obtained by performing conjugate multiplication processing on the channel state information of the two received signals obtained in each collecting period may be expressed as:
${H}_{cm} = \left[\begin{matrix}{H}_{11}^{1} conj \left({H}_{12}^{1}\right) \\ {H}_{11}^{2} conj \left({H}_{12}^{2}\right) \\ ⋮ \\ {H}_{11}^{L} conj \left({H}_{12}^{L}\right)\end{matrix}\right]$ (5)

Wherein *H_{cm}* represents the conjugate multiplication result matrix obtained by performing conjugate multiplication processing on the channel state information of the two received signals obtained in each collecting period.

Referring still to FIG. 1, in step S130: extracting amplitude information and phase information of the plurality of subcarriers from the plurality of conjugate multiplication result matrices, respectively.

Extracting amplitude information and phase information of the plurality of subcarriers from the plurality of conjugate multiplication result matrices, respectively, provides a basis for subsequently obtaining a plurality of corresponding living body detection training data based on the amplitude information and the phase information of the plurality of subcarriers, to form a living body detection training dataset.

Extracting amplitude information of the plurality of subcarriers from the plurality of conjugate multiplication result matrices, respectively, comprises: obtaining amplitude information correlation coefficient matrices of the plurality of subcarriers based on the conjugate multiplication result matrices, respectively.

In an embodiment of the present disclosure, a Wi-Fi transmitting device transmits a Wi-Fi signal using one transmitting antenna, and a Wi-Fi receiving device simultaneously receives the Wi-Fi signal transmitted by the Wi-Fi transmitting device using two receiving antennas. Correspondingly, extracting amplitude information of the plurality of subcarriers from the plurality of conjugate multiplication result matrices, respectively, comprises: obtaining amplitude information correlation coefficient matrices of the plurality of subcarriers based on the conjugate multiplication result matrices, respectively.

Specifically, the amplitude information correlation coefficient matrices of the plurality of subcarriers may be calculated using the following formula:
${R}_{am} = corr \left(\left‖{H}_{cm}\right‖, \left‖{{H}_{cm}}^{T}\right‖\right)$ (6)

Wherein, *Rₐₘ* represents the amplitude information correlation coefficient matrix of the plurality of subcarriers, *corr*(.) represents the correlation coefficient calculation operation, ∥*H*_{cm}∥ represents the amplitude calculation operation performed on the conjugate multiplication result matrix *H_{cm}*, ∥*H_{cm}^{T}*∥ represents the amplitude calculation operation performed on the transpose matrix *H_{cm}^{T}* of the conjugate multiplication result matrix.

Correspondingly, extracting phase information of the plurality of subcarriers from the plurality of conjugate multiplication result matrices, respectively, comprises: obtaining phase difference information correlation coefficient matrices and phase difference information correlation matrices of the plurality of subcarriers based on the conjugate multiplication result matrices, respectively.

Specifically, the phase difference information correlation coefficient matrices of the plurality of subcarriers may be calculated using the following formula:
${R}_{ph} = corr \left(∠{H}_{cm},∠{{H}_{cm}}^{T}\right)$ (7)

Wherein, *Rₚₕ* represents the phase difference information correlation coefficient matrix of the plurality of subcarriers, ∠*H*_{cm} represents the phase calculation operation performed on the conjugate multiplication result matrix *H_{cm}*, ∠*H_{cm}^{T}* represents the phase calculation operation performed on the transpose matrix *H_{cm}^{T}* of the conjugate multiplication result matrix *H_{cm}*.

Referring still to FIG. 1, in step S 140: obtaining a plurality of corresponding living body detection training data based on the amplitude information and the phase information of the plurality of subcarriers, to form a living body detection training dataset.

Obtaining a plurality of corresponding living body detection training data based on the amplitude information and the phase information of the plurality of subcarriers, to form a living body detection training dataset, provides a basis for subsequently performing learning training using the living body detection training data in the living body detection training dataset to obtain a corresponding living body detection model.

In an embodiment of the present disclosure, the living body detection training data comprises the following 15 features in 6 aspects:
(1) a living body respiration frequency value calculated based on a phase difference information correlation matrix of the plurality of subcarriers;
(2) a maximum eigenvalue, a second largest eigenvalue, and an entropy of eigenvalues after normalization of an amplitude information correlation coefficient matrix of the plurality of subcarriers;
(3) a maximum eigenvalue and a second largest eigenvalue after normalization of a phase difference information correlation coefficient matrix of the plurality of subcarriers;
(4) an energy value of a first principal component, an energy value of a second principal component, and an energy value of a third principal component, a ratio between a variance of the first principal component and a differential value of an eigenvector corresponding to the variance of the first principal component, a ratio between a variance of the second principal component and a differential value of an eigenvector corresponding to the variance of the second principal component, a ratio between a variance of the third principal component and a differential value of an eigenvector corresponding to the variance of the third principal component, and a kurtosis value of the second principal component, obtained by performing principal component analysis processing on the amplitude information of the plurality of subcarriers;
(5) a standard deviation of maximum eigenvalues after normalization of amplitude information correlation coefficient matrices of the plurality of subcarriers corresponding to a current collecting period and previous (N-1) collecting periods; and
(6) a standard deviation of second largest eigenvalues after normalization of phase difference information correlation coefficient matrices of the plurality of subcarriers corresponding to the current collecting period and the previous (N-1) collecting periods.

In a specific implementation, living bodies with different degrees of motion cause different variations among different subcarriers of each receiving antenna of the Wi-Fi receiving device, thereby affecting the maximum eigenvalue, the second largest eigenvalue, and the entropy of eigenvalues of the amplitude information correlation coefficient matrices of the plurality of subcarriers of each receiving antenna of the Wi-Fi receiving device, and simultaneously affecting the maximum eigenvalue and the second largest eigenvalue of the phase difference information correlation coefficient matrices of the plurality of subcarriers of each receiving antenna of the Wi-Fi receiving device, as well as the standard deviation of maximum eigenvalues after normalization of the amplitude information correlation coefficient matrices of the plurality of subcarriers corresponding to the current collecting period and the previous (N-1) collecting periods, and the standard deviation of second largest eigenvalues after normalization of the amplitude information correlation coefficient matrices of the plurality of subcarriers corresponding to the current collecting period and the previous (N-1) collecting periods.

Therefore, in an embodiment of the present disclosure, the maximum eigenvalue, the second largest eigenvalue, and the entropy of eigenvalues after normalization of the amplitude information correlation coefficient matrices of the plurality of subcarriers, the maximum eigenvalue and the second largest eigenvalue after normalization of the phase difference information correlation coefficient matrices of the plurality of subcarriers, as well as the standard deviation of maximum eigenvalues after normalization of the amplitude information correlation coefficient matrices of the plurality of subcarriers corresponding to the current collecting period and the previous (N-1) collecting periods, and the standard deviation of second largest eigenvalues after normalization of the amplitude information correlation coefficient matrices of the plurality of subcarriers corresponding to the current collecting period and the previous **(N-1)** collecting periods, are configured to serve as representations of dynamic features and static features of the living body.

**In** an embodiment of the present disclosure, the maximum eigenvalue, the second largest eigenvalue, and the entropy of eigenvalues after normalization of the amplitude information correlation coefficient matrices of the plurality of subcarriers may be expressed as follows, respectively:
${e}_{amm} = max \left(eigen\left({R}_{am}\right)/L\right)$ (8)
${e}_{ams} = s max \left(eigen\left({R}_{am}\right)/L\right)$ (9)
${E}_{entropy} = - \sum {P}_{i} log \left({P}_{i}\right)$ (10)

Wherein eₐₘₘ represents the maximum eigenvalue after normalization of the amplitude information correlation coefficient matrices of the plurality of subcarriers, eₐₘₛ represents the second largest eigenvalue after normalization of the amplitude information correlation coefficient matrices of the plurality of subcarriers, *eigen*(.) represents the operation of obtaining eigenvalues, max (.) represents the operation of taking the maximum value, s max (.) represents the operation of taking the second largest value, *E_{entropy}* represents the entropy of eigenvalues after normalization of the amplitude information correlation coefficient matrices of the plurality of subcarriers, *Pᵢ* represents the probability of the *i*-th eigenvalue after normalization of the amplitude information correlation coefficient matrices of the plurality of subcarriers, and log (.) represents the operation of taking the logarithm.

In an embodiment of the present disclosure, the maximum eigenvalue and the second largest eigenvalue after normalization of the phase difference information correlation coefficient matrices of the plurality of subcarriers may be expressed as follows, respectively:
${e}_{phm} = max \left(eigen\left({R}_{ph}\right)/L\right)$ (11)
${e}_{phs} = s max \left(eigen\left({R}_{ph}\right)/L\right)$ (12)

Wherein eₚₕₘ represents the maximum eigenvalue after normalization of the phase difference information correlation coefficient matrices of the plurality of subcarriers, and eₚₕₛ represents the second largest eigenvalue after normalization of the phase difference information correlation coefficient matrices of the plurality of subcarriers.

Based on practical needs, the value of N may be obtained by those skilled in the art based on the training needs of the living body detection model. As an example, a value range of N is from 5 times to 10 times.

In a specific implementation, different degrees of motion and stillness of living bodies located around the environment under detection, or different degrees of motion and stillness of living bodies passing through walls, cause changes in the first principal component, the second principal component, and the third principal component obtained by performing principal component analysis on the amplitude information of the plurality of subcarriers.

Correspondingly, the energy value of the first principal component, the energy value of the second principal component, and the energy value of the third principal component, the ratio between the variance of the first principal component and the differential value of the eigenvector corresponding to the variance of the first principal component, the ratio between the variance of the second principal component and the differential value of the eigenvector corresponding to the variance of the second principal component, the ratio between the variance of the third principal component and the differential value of the eigenvector corresponding to the variance of the third principal component, and the kurtosis value of the second principal component, obtained by performing principal component analysis (PCA) processing on the amplitude information of the plurality of subcarriers, are configured to serve as representations of dynamic features and static features of living bodies passing through walls.

Meanwhile, performing principal component analysis processing on the amplitude information of the plurality of subcarriers enables the realization of dimensionality reduction of the amplitude information of the plurality of subcarriers, and can filter out low-frequency noise to a certain extent.

In an embodiment of the present disclosure, the energy value of the [ordinal]-th principal component obtained by performing principal component analysis processing on the amplitude information of the plurality of subcarriers may be calculated using the following formula, respectively:
${g}_{i} = {\sum }_{k = 1}^{\frac{L}{2}} P {\left(k\right)}^{2}$ (13)
And:
$P \left(k\right) = \frac{FFT {\left(k\right)}^{2}}{{\sum }_{j = 1}^{L / 2} FFT {\left(j\right)}^{2}}$ (14)
${c}_{i} = {He}_{i}$ (15)
$E = eigen \left(C\right) = \left({e}_{0} ⋯ {e}_{S}\right)$ (16)
$C = corr \left(\overline{\left‖{{H}_{cm}}^{T}\right‖}, \overline{\left‖{H}_{cm}\right‖}\right)$ (17)

Wherein *gᵢ* represents the energy value of the *i*-th principal component obtained by performing principal component analysis processing on the amplitude information of the plurality of subcarriers, *FFT*(*k*) represents the value of the *k* -th frequency component after performing Fourier transform on the *i*-th principal component, cᵢ represents the *i*-th principal component obtained by performing principal component analysis processing on the amplitude information of the plurality of subcarriers, and ∥̅*̅H̅*̅_̅{̅c̅m̅}̅∥̅ represents the amplitude matrix of the conjugate multiplication result matrix *H_{cm}* with the direct current component removed.

In an embodiment of the present disclosure, the ratio between the variance of the *i*-th principal component and the differential value of the eigenvector corresponding to the variance of the -i th principal component is calculated using the following formula:
${e}_{i}^{r} = \frac{var \left({c}_{i}\right)}{{e}_{i}^{diff}}$ (18)
And:
${e}_{i}^{diff} = \frac{1}{S - 1} {\sum }_{k = 2}^{S} \left|{e}_{i}\left(k\right)-{e}_{i}\left(k-1\right)\right|$ (19)

Wherein ${e}_{i}^{r}$ represents the ratio between the variance of the *i*-th principal component and the differential value of the eigenvector corresponding to the variance of the *i*-th principal component, var (*cᵢ*) represents the variance calculation operation performed on the *i* -th principal component, and ${e}_{i}^{diff}$ represents the differential value of the eigenvector corresponding to the variance of the *i*-th principal component.

In an embodiment of the present disclosure, the kurtosis value of the second principal component is calculated using the following formula:
$z = \frac{\frac{1}{L} {\sum }_{i = 1}^{L} {\left({c}_{2}-\overline{{c}_{2}}\right)}^{4}}{{\left(\frac{1}{L}{\left({c}_{2}-\overline{{c}_{2}}\right)}^{2}\right)}^{2}}$ (20)

Wherein, z represents the kurtosis value of the second principal component, *c*₂ represents the second principal component, *̅c̅*̅₂̅ represents the mean value of the second principal component.

It should be noted that when a stationary human body exists in the environment under detection, the dynamic and static features within the environment under detection are very similar to the dynamic and static features of living bodies passing through walls. In this case, the living body respiration frequency value calculated based on the phase difference information correlation matrix of the plurality of subcarriers can be used to distinguish an environment with a stationary living body from an environment that is stationary and unoccupied.

In an embodiment of the present disclosure, the living body respiration frequency value is calculated based on the phase difference information correlation matrix of the plurality of subcarriers using a Multiple Signal Classification (MUSIC) algorithm.

In an embodiment of the present disclosure, the phase difference information correlation matrix of the plurality of subcarriers is calculated using the following formula:
$R = \overline{∠ {H}_{cm}} ∗ {\overline{∠ {H}_{cm}}}^{T}$ (21)

Wherein *R* represents the phase difference information correlation matrix of the plurality of subcarriers, ∠̅*̅H̅*̅_̅{̅c̅m̅}̅ represents the phase matrix of the conjugate multiplication result matrix *H_{cm}* with the direct current component removed, and ∠̅*̅H̅*̅_̅{̅c̅m̅}̅*^{T}* represents the transpose matrix of the phase matrix ∠̅*̅H̅*̅_̅{̅c̅m̅}̅ of the conjugate multiplication result matrix *H_{cm}* with the direct current component removed.

In some other embodiments of the present disclosure, the living body respiration frequency value can also be calculated based on the phase difference information correlation matrix of the plurality of subcarriers using other super-resolution algorithms. The other super-resolution algorithms comprise a root Multiple Signal Classification (root_MUSIC) algorithm, a Minimum Variance Distortionless Response (MVDR) algorithm, and an Estimation of Signal Parameters using Rotational Invariance Techniques (ESPRIT) algorithm, or the like.

When calculating the living body respiration frequency value, the sliding step size is the collecting period; in other words, the living body respiration frequency value is calculated once per collecting period, and the time length of the sliding time window for calculating the living body respiration frequency value is related to the respiration frequency of the living body. Specifically, the time length of the sliding time window shall be greater than or equal to the time length of one respiration of the living body, and shall be an integer multiple of the time length of one respiration of the living body.

In an embodiment of the present disclosure, in order to simultaneously satisfy the needs of Wi-Fi device communication and living body detection, the living body detection model is configured to perform human body detection. The respiration frequency of a human body is generally from 0.1 Hz to 0.6 Hz. Therefore, in the present embodiment, the time length of the sliding time window is from 1.7s to 10s.

In some other embodiments of the present disclosure, the living body detection model can also be configured to perform human body detection of other animal bodies having a respiration frequency close to the respiration frequency of the human body. The other animal bodies having a respiration frequency close to the respiration frequency of the human body comprise cats, dogs, rabbits, pigs, horses, cattle, sheep, or the like.

Referring still to FIG. 1, in step S150: performing learning training using the living body detection training data in the living body detection training dataset to obtain a corresponding living body detection model.

Performing learning training using the living body detection training data in the living body detection training dataset to obtain a corresponding living body detection model, such that the living body detection model can be used for living body detection.

In an embodiment of the present disclosure, performing learning training using the human body detection training data in the human body detection training dataset to obtain a corresponding human body detection model.

In some embodiments of the present disclosure, the living body detection model comprises a support vector machine (SVM) classifier. Correspondingly, performing learning training on the living body detection training data in the living body detection training dataset using a support vector machine algorithm to obtain a corresponding living body detection model.

In some other embodiments of the present disclosure, the living body detection model can also be other classifiers, such as an Adaptive Boosting (Adaboost) classifier, a Bayes classifier, a Back Propagation (BP) neural network classifier, or the like, or can also be other feature classification models, which are not limited herein.

To address the above technical problem, the embodiments of the present disclosure further provide a generation module for a living body detection model.

FIG. 2 shows a schematic structural diagram of a generation module for a living body detection model provided by an embodiment of the present disclosure. Referring to FIG. 2, a generation module 200 for a living body detection model, comprising: a first obtaining sub-module 201, configured to obtain channel state information of multi-channel received signals of a Wi-Fi receiving device based on a preset collecting period, a received signal of each channel comprising a plurality of subcarriers; a conjugate multiplication sub-module 202, configured to perform conjugate multiplication processing on the channel state information of any two of the multi-channel received signals obtained in each collecting period, respectively, to obtain a plurality of corresponding conjugate multiplication result matrices; an information extraction sub-module 203, configured to extract amplitude information and phase information of the plurality of subcarriers from the plurality of conjugate multiplication result matrices, respectively; a data obtaining sub-module 204, configured to obtain a plurality of corresponding living body detection training data based on the amplitude information and the phase information of the plurality of subcarriers, to form a living body detection training dataset; and a model training sub-module 205, configured to perform learning training using the living body detection training data in the living body detection training dataset to obtain a corresponding living body detection model.

The generation module for a living body detection model in the embodiments of the present disclosure can be configured to execute the aforementioned generation method for a living body detection model, or other functional modules can also be configured to execute the aforementioned generation method for a living body detection model. For the generation method for a living body detection model, please refer to the detailed description in the foregoing section, and details are not described herein again.

To address the above technical problem, the embodiments of the present disclosure further provide a living body detection method.

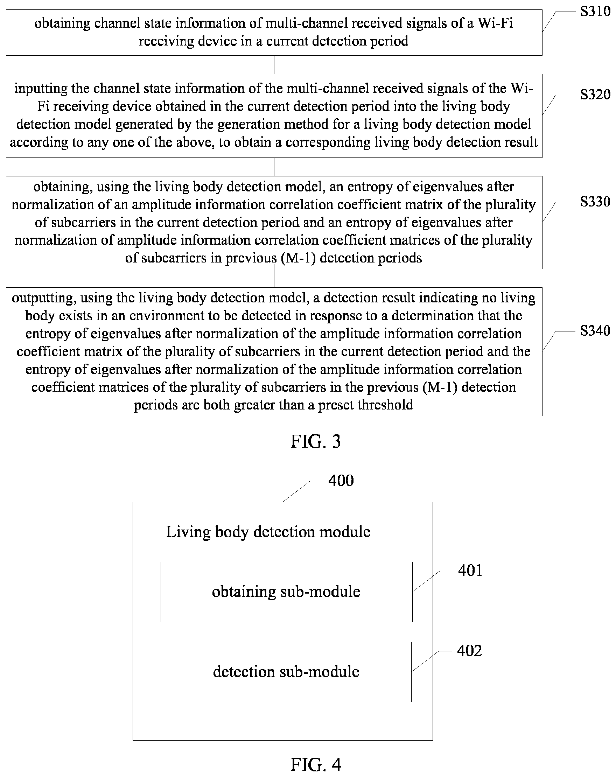

FIG. 3 shows a schematic flow diagram of a living body detection method provided by an embodiment of the present disclosure. Referring to FIG. 3, a living body detection method may specifically comprise the following steps:

Step S310: obtaining channel state information of multi-channel received signals of a Wi-Fi receiving device in a current detection period; and

Step S320: inputting the channel state information of the multi-channel received signals of the Wi-Fi receiving device obtained in the current detection period into the living body detection model generated by the generation method for a living body detection model according to any one of the above, to obtain a corresponding living body detection result.

In some embodiments, inputting the channel state information of the multi-channel received signals of the Wi-Fi receiving device obtained in the current detection period into the living body detection model generated by the generation method for a living body detection model according to any one of the above, such that the living body detection model can extract the 15 features in the 6 aspects mentioned in step S 140, and determine, based on the extracted features, a detection result indicating whether a living body exists in the environment to be detected.

In an embodiment of the present disclosure, the living body detection model is configured to perform human body detection. Correspondingly, inputting the channel state information of the multi-channel received signals of the Wi-Fi receiving device obtained in the current detection period into the human body detection model, such that the human body detection model can extract the 15 features in the 6 aspects mentioned in step S140, and determine, based on the extracted features, a detection result indicating whether a human body exists in the environment to be detected.

In some other embodiments of the present disclosure, the living body detection model can also be configured to perform human body detection of other animal bodies having a respiration frequency close to the respiration frequency of the human body.

In an embodiment of the present disclosure, the living body detection method further comprising:

Step S330: obtaining, using the living body detection model, an entropy of eigenvalues after normalization of an amplitude information correlation coefficient matrix of the plurality of subcarriers in the current detection period and an entropy of eigenvalues after normalization of amplitude information correlation coefficient matrices of the plurality of subcarriers in previous (M-1) detection periods; and

Step S340: outputting, using the living body detection model, a detection result indicating no living body exists in an environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold.

Using the living body detection model to output a detection result indicating no living body exists in the environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold, enables the avoidance of the influence of living bodies of neighbors on the living body detection of the environment to be detected, prevents false alarms, and accordingly helps further improve the accuracy of living body detection.

The value of M may be set based on the needs of living body detection. As an example, a value range of M is from 5 times to 10 times.

To address the above technical problem, the embodiments of the present disclosure further provide a living body detection module.

FIG. 4 shows a schematic structural diagram of a living body detection module provided by an embodiment of the present disclosure. Referring to FIG. 4, a living body detection module 400, comprising: an obtaining sub-module 401, configured to obtain channel state information of multi-channel received signals of a Wi-Fi receiving device in a current detection period; and a detection sub-module 402, configured to input the channel state information of the multi-channel received signals of the Wi-Fi receiving device obtained in the current detection period into the living body detection model generated by the generation method for a living body detection model provided by the embodiments of the present disclosure, to obtain a corresponding living body detection result.

The living body detection module in the embodiments of the present disclosure can be configured to execute the aforementioned living body detection method, or other functional modules can also be configured to execute the aforementioned living body detection method. For the living body detection method, please refer to the detailed description in the foregoing section, and details are not described herein again.

To address the above technical problem, the embodiments of the present disclosure further provide a chip, wherein the generation module for a living body detection model or the living body detection module according to the embodiments of the present disclosure is integrated on the chip. For the generation module for a living body detection model or the living body detection module, please refer to the detailed description in the foregoing section, and details are not described herein again.

To address the above technical problem, the embodiments of the present disclosure further provide a storage medium, wherein the storage medium stores one or more computer instructions, and the one or more computer instructions are configured to implement the generation method for a living body detection model or the living body detection method according to the embodiments of the present disclosure. For the generation method for a living body detection model or the living body detection method, please refer to the detailed description in the foregoing section, and details are not described herein again.

To address the above technical problem, the embodiments of the present disclosure further provide an electronic device, comprising at least one memory and at least one processor, wherein the memory stores one or more computer instructions, and the one or more computer instructions are executed by the processor to implement the generation method for a living body detection model or the living body detection method according to the embodiments of the present disclosure. For the generation method for a living body detection model or the living body detection method, please refer to the detailed description in the foregoing section, and details are not described herein again.

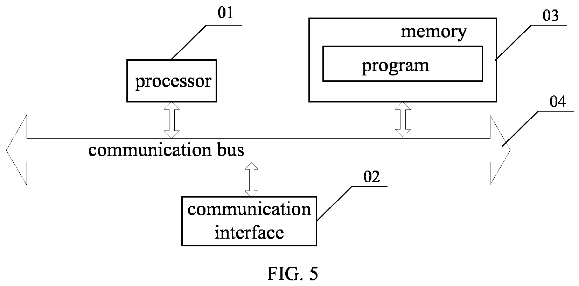

An optional hardware structure of the electronic device provided by an embodiment of the present disclosure may be as shown in FIG. 5, comprising: at least one processor 01, at least one communication interface 02, at least one memory 03, and at least one communication bus 04.

In the embodiments of the present disclosure, the number of the processor 01, the communication interface 02, the memory 03, and the communication bus 04 is at least one, and the processor 01, the communication interface 02, and the memory 03 communicate with each other through the communication bus 04.

The communication interface 02 may be an interface of a communication module configured to perform network communication, such as an interface of a GSM module.

The processor 01 may be a central processing unit (CPU), an Application Specific Integrated Circuit (ASIC), or one or more integrated circuits configured to implement the embodiments of the present disclosure.

The memory 03 may comprise a high-speed RAM memory, and may also comprise a non-volatile memory, such as at least one disk memory.

The memory 03 stores one or more computer instructions, and the one or more computer instructions are executed by the processor 01 to implement the generation method for a living body detection model or the living body detection method according to the embodiments of the present disclosure.

It should be noted that the aforementioned terminal device may also comprise other components not shown that may not be necessary for understanding the embodiments of the present disclosure; since these other components may not be necessary for understanding the embodiments of the present disclosure, the embodiments of the present disclosure do not introduce them one by one.

An embodiment of the present disclosure further provides a storage medium, wherein the storage medium stores one or more computer instructions, and the one or more computer instructions are configured to implement the generation method for a living body detection model or the living body detection method according to the embodiments of the present disclosure.

The above-described embodiments of the present disclosure are combinations of elements and features of the present disclosure. Unless otherwise mentioned, the elements or features may be considered optional. Each element or feature may be practiced without combining with other elements or features. **In** addition, the embodiments of the present disclosure may be constructed by combining some elements and/or features. The order of operations described in the embodiments of the present disclosure may be rearranged. Some configurations of any one embodiment may be included in another embodiment, and may be replaced by corresponding configurations of another embodiment. **It** will be apparent to those skilled in the art that claims in the appended claims that do not have explicit citation relationships with each other may be combined into embodiments of the present disclosure, or may be included as new claims in amendments filed after the submission of the present application.

The embodiments of the present disclosure may be implemented by various means such as hardware, firmware, software, or a combination thereof as an example. **In a** hardware configuration, the method provided by the embodiments of the present disclosure may be implemented by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, microcontrollers, microprocessors, or the like.

**In a** firmware or software configuration, the embodiments of the present disclosure may be implemented in the form of modules, procedures, functions, or the like. Software code may be stored in a memory unit and executed by a processor. The memory unit may be located inside or outside the processor, and may send data to and receive data from the processor via various known means.

The above description of the disclosed embodiments enables those skilled in the art to make or use the present disclosure. Various modifications to these embodiments will be apparent to those skilled in the art, and the general principles defined herein may be implemented in other embodiments without departing from the spirit or scope of the present disclosure. Therefore, the present disclosure will not be limited to the embodiments shown herein, but is to be accorded the widest scope consistent with the principles and novel features disclosed herein.

Although the embodiments of the present disclosure are disclosed as above, the present disclosure is not limited thereto. Any person skilled in the art may make various changes and modifications without departing from the spirit and scope of the present disclosure, and therefore the scope of protection of the present disclosure shall be subject to the scope defined by the claims.

## Claims

1. A generation method for a living body detection model, comprising:
obtaining channel state information of multi-channel received signals of a Wi-Fi receiving device based on a preset collecting period, a received signal of each channel comprising a plurality of subcarriers;
performing conjugate multiplication processing on the channel state information of any two of the multi-channel received signals obtained in each collecting period, respectively, to obtain a plurality of corresponding conjugate multiplication result matrices;
extracting amplitude information and phase information of the plurality of subcarriers from the plurality of conjugate multiplication result matrices, respectively;
obtaining a plurality of corresponding living body detection training data based on the amplitude information and the phase information of the plurality of subcarriers, to form a living body detection training dataset; and
performing learning training using the living body detection training data in the living body detection training dataset to obtain a corresponding living body detection model.

2. The generation method for a living body detection model of claim 1, wherein the living body detection training data comprises:
a living body respiration frequency value calculated based on a phase difference information correlation matrix of the plurality of subcarriers;
a maximum eigenvalue, a second largest eigenvalue, and an entropy of eigenvalues after normalization of an amplitude information correlation coefficient matrix of the plurality of subcarriers;
an energy value of a first principal component, an energy value of a second principal component, and an energy value of a third principal component, a ratio between a variance of the first principal component and a differential value of an eigenvector corresponding to the variance of the first principal component, a ratio between a variance of the second principal component and a differential value of an eigenvector corresponding to the variance of the second principal component, a ratio between a variance of the third principal component and a differential value of an eigenvector corresponding to the variance of the third principal component, and a kurtosis value of the second principal component, obtained by performing principal component analysis processing on the amplitude information of the plurality of subcarriers;
a maximum eigenvalue and a second largest eigenvalue after normalization of a phase difference information correlation coefficient matrix of the plurality of subcarriers;
a standard deviation of maximum eigenvalues after normalization of amplitude information correlation coefficient matrices of the plurality of subcarriers corresponding to a current collecting period and previous (N-1) collecting periods; and
a standard deviation of second largest eigenvalues after normalization of phase difference information correlation coefficient matrices of the plurality of subcarriers corresponding to the current collecting period and the previous (N-1) collecting periods.

3. The generation method for a living body detection model of claim 2, wherein a value range of N is from 5 times to 10 times.

4. The generation method for a living body detection model of claim 1, wherein a time length of the collecting period is from 1s to 3s.

5. The generation method for a living body detection model of claim 1, wherein the living body detection model comprises a support vector machine classifier.

6. The generation method for a living body detection model of any one of claims 1 to 5, wherein the living body comprises at least one of a human body and an animal body having a respiration frequency close to the respiration frequency of the human body.

7. A generation module for a living body detection model, comprising:
a first obtaining sub-module, configured to obtain channel state information of multi-channel received signals of a Wi-Fi receiving device based on a preset collecting period, a received signal of each channel comprising a plurality of subcarriers;
a conjugate multiplication sub-module, configured to perform conjugate multiplication processing on the channel state information of any two of the multi-channel received signals obtained in each collecting period, respectively, to obtain a plurality of corresponding conjugate multiplication result matrices;
an information extraction sub-module, configured to extract amplitude information and phase information of the plurality of subcarriers from the plurality of conjugate multiplication result matrices, respectively;
a data obtaining sub-module, configured to obtain a plurality of corresponding living body detection training data based on the amplitude information and the phase information of the plurality of subcarriers, to form a living body detection training dataset; and
a model training sub-module, configured to perform learning training using the living body detection training data in the living body detection training dataset to obtain a corresponding living body detection model.

8. The generation module for a living body detection model of claim 7, wherein the living body detection training data obtained by the data obtaining sub-module comprises:
a living body respiration frequency value calculated based on a phase difference information correlation matrix of the plurality of subcarriers;
a maximum eigenvalue, a second largest eigenvalue, and an entropy of eigenvalues after normalization of an amplitude information correlation coefficient matrix of the plurality of subcarriers;
an energy value of a first principal component, an energy value of a second principal component, and an energy value of a third principal component, a ratio between a variance of the first principal component and a differential value of an eigenvector corresponding to the variance of the first principal component, a ratio between a variance of the second principal component and a differential value of an eigenvector corresponding to the variance of the second principal component, a ratio between a variance of the third principal component and a differential value of an eigenvector corresponding to the variance of the third principal component, and a kurtosis value of the second principal component, obtained by performing principal component analysis processing on the amplitude information of the plurality of subcarriers;
a maximum eigenvalue and a second largest eigenvalue after normalization of a phase difference information correlation coefficient matrix of the plurality of subcarriers;
a standard deviation of maximum eigenvalues after normalization of amplitude information correlation coefficient matrices of the plurality of subcarriers corresponding to a current collecting period and previous (N-1) collecting periods; and
a standard deviation of second largest eigenvalues after normalization of phase difference information correlation coefficient matrices of the plurality of subcarriers corresponding to the current collecting period and the previous (N-1) collecting periods.

9. The generation module for a living body detection model of claim 7, wherein a value range of N is from 5 times to 10 times.

10. The generation module for a living body detection model of claim 6, wherein a time length of the collecting period is from 1s to 3s.

11. The generation module for a living body detection model of claim 6, wherein the living body detection model comprises a support vector machine classifier.

12. The generation module for a living body detection model of any one of claims 7 to 11, wherein the living body comprises at least one of a human body and an animal body having a respiration frequency close to the respiration frequency of the human body.

13. A living body detection method, comprising:
obtaining channel state information of multi-channel received signals of a Wi-Fi receiving device in a current detection period; and
inputting the channel state information of the multi-channel received signals of the Wi-Fi receiving device obtained in the current detection period into the living body detection model generated by the generation method for a living body detection model of any one of claims 1 to 6, to obtain a corresponding living body detection result.

14. The living body detection method of claim 13, wherein the received signal of each channel comprises a plurality of subcarriers;
the living body detection method further comprising:
obtaining, using the living body detection model, an entropy of eigenvalues after normalization of an amplitude information correlation coefficient matrix of the plurality of subcarriers in the current detection period and an entropy of eigenvalues after normalization of amplitude information correlation coefficient matrices of the plurality of subcarriers in previous (M-1) detection periods; and
outputting, using the living body detection model, a detection result indicating no living body exists in an environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold.

15. The living body detection method of claim 14, wherein a value range of M is from 5 times to 10 times.

16. A living body detection module, comprising:
an obtaining sub-module, configured to obtain channel state information of multi-channel received signals of a Wi-Fi receiving device in a current detection period; and
a detection sub-module, configured to input the channel state information of the multi-channel received signals of the Wi-Fi receiving device obtained in the current detection period into the living body detection model generated by the generation method for a living body detection model of any one of claims 1 to 6, to obtain a corresponding living body detection result.

17. The living body detection module of claim 16, wherein the received signal of each channel comprises a plurality of subcarriers;
the detection sub-module is further configured to obtain, using the living body detection model, an entropy of eigenvalues after normalization of an amplitude information correlation coefficient matrix of the plurality of subcarriers in the current detection period and an entropy of eigenvalues after normalization of amplitude information correlation coefficient matrices of the plurality of subcarriers in previous (M-1) detection periods; and output, using the living body detection model, a detection result indicating no living body exists in an environment to be detected in response to a determination that the entropy of eigenvalues after normalization of the amplitude information correlation coefficient matrix of the plurality of subcarriers in the current detection period and the entropy of eigenvalues after normalization of the amplitude information correlation coefficient matrices of the plurality of subcarriers in the previous (M-1) detection periods are both greater than a preset threshold.

18. The living body detection module of claim 17, wherein a value range of M is from 5 times to 10 times.

19. A chip, wherein the generation module for a living body detection model of any one of claims 7 to 12 or the living body detection module of any one of claims 16 to 18 is integrated on the chip.

20. An electronic device, comprising at least one memory and at least one processor, wherein the memory stores one or more computer instructions, and the one or more computer instructions are executed by the processor to implement the generation method for a living body detection model of any one of claims 1 to 6 or the living body detection method of any one of claims 13 to 15.

21. A storage medium, wherein the storage medium stores one or more computer instructions, and the one or more computer instructions are configured to implement the generation method for a living body detection model of any one of claims 1 to 6 or the living body detection method of any one of claims 13 to 15.
